(19) Europäisches Patentamt
European Patent Office
Office européen des brevets

(11) Publication number: 0 083 121
B1

# (12) EUROPEAN PATENT SPECIFICATION

(45) Date of publication of patent specification: 24.07.85

(51) Int. Cl.[4]: C 07 C 51/12, C 07 C 67/36, C 07 C 67/37, B 01 J 31/28

(21) Application number: 82201542.6

(22) Date of filing: 02.12.82

## (54) Process for the co-production of carboxylic acids and carboxylic acid esters.

(30) Priority: 10.12.81 GB 8137359
19.07.82 GB 8220848

(43) Date of publication of application:
06.07.83 Bulletin 83/27

(45) Publication of the grant of the patent:
24.07.85 Bulletin 85/30

(84) Designated Contracting States:
BE DE FR GB IT NL

(56) References cited:
EP-A-0 022 038
EP-A-0 031 606
EP-A-0 046 129
DE-B-2 303 271
GB-A-2 068 377

(73) Proprietor: SHELL INTERNATIONALE RESEARCH MAATSCHAPPIJ B.V.
Carel van Bylandtlaan 30
NL-2596 HR Den Haag (NL)

(72) Inventor: Drent, Eit
Badhuisweg 3
NL-1031 CM Amsterdam (NL)

(74) Representative: Aalbers, Onno et al
P.O. Box 302
NL-2501 CH The Hague (NL)

EP 0 083 121 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the co-production of carboxylic acids and carboxylic acid esters from carboxylic acid esters having one carbon atom less in the molecule, carbon monoxide and hydrogen in the presence of a catalytic system. The invention relates in particular to a process for the co-production of acetic acid and ethyl acetate from methyl acetate under mild process conditions. Carboxylic acid esters produced according to the process according to the present invention are thus homologues of the carboxylic acid esters used as starting materials.

The production of carboxylic acid esters via homologation has already been described in the literature.

It is known from European Patent Application No. 31606 that the stoichiometry of the known reactions of methyl acetate with carbon monoxide and hydrogen can be altered most advantageously to produce one mole of ethyl acetate and two moles of acetic acid from two moles of methyl acetate. The catalytic system comprises three metal compounds: a ruthenium compound, a further Group VIII metal compound, and a bromide or iodide of a Group II or transition metal, preferably in the presence of a promoter, typically an amine or a phosphine.

It has now been found that compounds of penta-valent phosphorus, arsenic or antimony having the general formula I indicated below can also be applied advantageously as very active promotors in the process for the co-production of carboxylic acids and carboxylic acid esters using a catalytic system based on a ruthenium compound, a further Group VIII metal compound and an alkyl or acyl iodide or bromide. It has been found that the activity of metal catalyst is strongly increased compared with the catalyst used in the process of the prior art.

The present invention provides a process for the co-production of carboxylic acids of the general formula $R^1$—COOH and $R^2$—COOH and carboxylic acid esters of the general formula $R^1$—$COOCH_2R^2$ and $R^2$—$COOCH_2R^1$ wherein each of the groups $R^1$ and $R^2$, which may be the same or different, represents an alkyl group having from 1 to 20 carbon atoms which may be substituted by fluorine or chlorine, or an aryl, alkaryl or aralkyl group which may be substituted by fluorine or chlorine whilst $R^1$ may also represent a hydrogen atom, wherein a carboxylic acid ester of the general formula $R^1$—$COOR^2$ and/or an ether of the general formula $R^3OR^4$, wherein $R^1$ and $R^2$ are as defined hereinbefore and each of $R^3$ and $R^4$, which may be the same or different, represents an alkyl group having from 1 to 20 carbon atoms which may be substituted by fluorine or chlorine, or an aryl, alkaryl or aralkyl group which may be substituted by fluorine or chlorine, is reacted with carbon monoxide and hydrogen at elevated temperature and pressure characterised in that the reaction is carried out in the presence of a catalytic system which comprises a ruthenium compound, a further Group VIII metal compound, a compound of the formula $R^5$Hal or $R^5$COHal, wherein $R^5$ has one of the meanings given above for $R^2$ and Hal represents an iodide or bromide atom, and a compound having the formula:

$$YZ[(O)_aR'][(O)_bR''][(O)_cR'''] \qquad \text{I}$$

wherein Y represents oxygen, sulphur or selenium, Z represents phosphorus, arsenic or antimony, each of R′, R″ and R‴ independently represents a hydrogen atom or an optionally substituted alkyl, cycloalkyl or aryl group or R′ has this meaning and R″ and R‴ together represent an alkylene group, and each of a, b and c independently represents 0 or 1, the reaction mixture being substantially free from other transition metal or Group II iodides or bromides.

It should be noted that the composition of the reaction product mixture will be governed by the choice of the starting carboxylic acid esters and/or ethers. For instance, when starting materials are used wherein the groups $R^1$ and $R^2$ are identical, such as in methyl acetate, dimethyl ether and ethyl propionate, the reaction product mixture will normally contain only the carboxylic acid ester homologue and the appropriate acid. When starting materials are used wherein the groups $R^1$ and $R^2$ are not identical, a more complex reaction product mixture will be obtained which comprises normally at least two carboxylic acid ester homologues and two appropriate carboxylic acids. For instance, when ethyl acetate is used as the starting material the reaction product mixture comprises propyl acetate, ethyl propionate, propionic acid and acetic acid.

It will be appreciated that any carboxylic acid ester homologue produced according to the present process can serve as starting material in the process according to the present invention thus forming the next carboxylic acid ester homologue(s) and the appropriate carboxylic acid(s). In addition, since carboxylic acids are produced in the process according to the present invention, transesterification reactions, i.e. reactions between carboxylic acids and carboxylic acid esters, or between different carboxylic acid esters, may also occur under the prevailing reaction conditions. It will be clear that transesterification reactions do not alter the product composition when the starting material comprises compounds wherein $R^1$ and $R^2$ are identical, but may alter the product composition when the groups $R^1$ and $R^2$ are not identical.

For the purpose of the present invention, carboxylic acids and carboxylic acid esters, obtained via a further homologation of produced carboxylic acid ester, or obtained by a transesterification process under the prevailing conditions, are considered to be within the scope of the present invention.

From the above it will be clear that preference is given to processes wherein starting materials are used wherein the groups $R^1$ and $R^2$ are identical since a less complex reaction mixture will be obtained. The

process according to the present invention is of special interest for the co-production of acetic acid and ethyl acetate from methyl acetate according to the equation:

$$2CH_3COOCH_3+2CO+2H_2 \rightarrow CH_3COOC_2H_5+2CH_3COOH$$

since the products can be obtained with high selectivity and close to the stoichiometrically expected ratio. This is of special interest when the process according to the invention is part of an integrated process, wherein acid produced—for instance acetic acid—is to be recycled in the process. Moreover, the process according to the present invention can be carried out conveniently at surprisingly low pressures, e.g. pressures well below 100 bar can be used advantageously.

Suitable starting materials which can be used conveniently in the process according to the present invention include compounds of the general formula $R^1$—$COOR^2$ and/or $R^3OR^4$, wherein each of $R^1$, $R^2$, $R^3$ and $R^4$, which may be the same or different, represents an alkyl group having from 1 to 12 carbon atoms, or an aryl, alkaryl or aralkyl group having up to 12 carbon atoms, whilst $R^1$ may also represent a hydrogen atom. Preference is given to the use of compounds of the general formula $R^1$—$COOR^2$ and/or $R^3OR^4$, wherein $R^1$, $R^2$, $R^3$ and $R^4$ are the same and each represents an alkyl group having from 1 to 12 carbon atoms or an aryl, alkaryl or aralkyl group having up to 12 carbon atoms. Most preferred starting materials are methyl acetate and dimethyl ester.

When ethers of the general formula $R^3OR^4$ are used as starting materials in the process according to the present invention, it would appear that these compounds will be converted primarily into the corresponding esters by the introduction of a carbon monoxide moiety into the molecule which molecule may then undergo the homologation reaction according to the present invention. If desired, the reaction according to the present invention may be out in two stages when an ether is used as the starting material. Firstly, the ether is converted into the corresponding ester which in its turn, in the same or in a different vessel, is converted into the final products. If desired, mixtures of carboxylic acid esters and/or ethers can be used as starting materials.

Ruthenium compounds which can be used conveniently in the process according to the present invention include ruthenium (III) chloride, ruthenium (III) chloride trihydrate, ruthenium (IV) chloride, ruthenium (III) bromide, the ruthenium oxides, organic ruthenium salts such as ruthenium (III) propionate, ruthenium (III) butyrate, ruthenium pentacarbonyl, trirutheniumdodecacarbonyl and mixed ruthenium halocarbonyls such as bis-(rutheniumtricarbonyl-dibromide), and other organo-ruthenium complexes.

Further Group VIII metal compounds which can be used together with a ruthenium compound in the catalytic system include palladium and, especially, rhodium compounds, although other Group VIII metal compounds can also be used. Examples of suitable rhodium compounds include rhodium oxide, rhodium (III) hydroxide, rhodium (III) chloride, rhodium (III) chloride trihydrate, rhodium (III) bromide, rhodium (III) iodide and the corresponding pyridine and phosphine complexes such as tris(pyridine) rhodium (III) chloride or dichloro bis(triphenylphosphine) rhodium, rhodium (III) formate, rhodium (III) acetate, rhodium (III) butyrate, rhodium (III) naphthenate, dirhodium octacarbonyl tetrarhodium dodecarbonyl, hexarhodium hexadecacarbonyl, rhodium, dicarbonylacetylacetonate and other organo-rhodium complexes. Preference is given to the use of rhodium (III) chloride trihydrate.

Examples of suitable palladium compounds include palladium chloride, palladium chloride dihydrate, palladium bromide, palladium iodide, palladium oxide, or an organic palladium salt or complex such as palladium formate, palladium acetate, palladium butyrate and palladium acetylacetonate. Preferred palladium compounds are palladium chloride, palladium chloride dihydrate and palladium acetate.

The molar ratio of ruthenium compound to further Group VIII metal compound is not critical and can vary between wide limits, e.g. atomic ratios of ruthenium to further Group VIII metal between 50:1 and 1:20, especially 10:1 and 1:5, are suitable.

The amount of ruthenium compound and further Group VIII metal compound to be used is not critical and any amount which exerts catalytic activity can be used. Amounts as low as 0.001%w, calculated on carboxylic acid ester or ether to be converted can be used, preference being given to amounts in the range of from 0.1—10%w, most preferably between 0.05—5%w.

Any iodide or bromide $R^5Hal$ $R^5COHal$ may be used in the process according to the present invention, but preferably $R^5$ has one of the preferred meanings given above for $R^2$, and preferably the group $R^5$ is identical to one of the groups $R^1$, $R^2$, $R^3$ or $R^4$ in the starting material, as this avoids the formation of additional mixed products. Especially preferred is the use of a reaction mixture in which $R^1$ and $R^2$ are the same, and the iodide or bromide has the formula $R^2I$, $R^2Br$, $R^2COI$ or $R^2COBr$. Thus for example when using the preferred feedstocks methyl acetate and/or dimethyl ether, methyl iodide or bromide or acetyl iodide or bromide, or any mixture thereof, is preferably used.

The quantity of iodide or bromide added to the reaction mixture is not crucial. Suitably the number of moles of added iodide plus bromide per gram atom of total Group VIII metal is in the range of from 0.1:1 to 200:1, preferably 1:1 to 100:1, and especially 10:1 to 50:1.

As stated hereinbefore, the process according to the present invention is carried out in the presence of a compound having the formula

$$YZ[(O)_aR'][(O)_bR''][(O)_cR''']. $$

Preferably any optionally substituted alkyl, cycloalkyl or aryl group represented by R′, R″ and/or R‴ has up to 20, more preferably up to 7 carbon atoms. The substituents may be any substituent inert under the reaction conditions, for example halogen atoms. Preferably each of a, b and c is 0.

The compounds having the general formula I may be oxides, sulphides or selenides of tertiary phosphines, arsines or stibines such as for example the oxides, sulphides or selenides or trimethylphosphine, triethylphosphine, tri-n-butylphosphine, triphenylphosphine, tri-p-tolylphosphine, tricyclohexylphosphine, diphenylethylphosphine and tris(1-naphthyl)phosphine and their arsenic or antimony analogues. The compound having formula I may contain two or more phosphorus, arsenic or antimony atoms. For example group R′ may be substituted with one or more groups —YZR″R‴. Examples of compounds of this type are tetraphenyldimethylene diphosphine dioxide (diphosdioxide) and tetraphenyl trimethylene diphosphine dioxide. Preference is given to the use of triphenyl phosphine oxide or triphenyl phosphine sulphide.

Alternatively the compounds having formula I may be, for example, the alkyl and aryl esters of phosphoric, phosphonic and phosphinic acids and their arsenic or antimony analogues. Examples of such compounds are trimethylphosphate, triethylphosphate, tri-n-butylphosphate, triphenylphosphate, dimethyl methylphosphonate, diethyl methylphosphonate, diphenyl methylphosphonate, methyl dimethylphosphinate, methyl diethylphosphinate and methyl diphenylphosphinate.

It will be appreciated that in the reaction mixture complexes are formed by the reaction of the oxide, sulphide or selenide of the phosphine, arsine or stibine with the iodine or bromine compound present or HBr of HI formed therefrom in situ. Examples of such complexes are

$$[(C_2H_5)_3AsO—H—OAs(C_2H_5)_3^+I^-$$

or

$$[(C_6H_5)_3PO—H—OP(C_6H_5)_3]^+I_3^-.$$

Consequently, the use of such complexes when prepared separately, is within the scope of the present invention.

Furthermore, it will be appreciated that the compound of penta-valent phosphorus, arsenic or antimony having the formula I can be formed in situ. For example, a phosphine oxide can be formed in situ from a phosphine by carrying out the reaction in the presence of molecular oxygen.

The molar ratio of the compounds having formula I in which Y represents an oxygen atom, to ruthenium is preferably in the range of from 0.01:1 to 20:1, preferably in the range of from 0.05:1 to 10:1. When in the compound having formula I the group Y represents sulphur or selenium and the groups R′, R″ and R‴ are alkyl, cycloalkyl or aryl groups not substituted with electron-attracting groups, the reaction mixture should preferably contain 0.01—1, more preferably 0.05—0.5, most preferably 0.05—0.3 mol of said compound per gram atom of ruthenium.

The process according to the present invention can be carried out using a wide range of temperatures. Temperatures up to 300°C can be suitably applied. Preference is given to temperatures in the range of from 50°C to 200°C, most preferred temperatures are in the range between 125°C and 175°C.

The process according to the present invention can be carried out using low pressures, e.g. pressures as low as 5 bar. Pressures in the range of from 20 to 100 bar are preferred. Higher pressures, e.g. pressures as high as 1000 bar can be applied, but they are generally not economical because of the investment and energy costs involved.

According to the reaction equation carbon monoxide and hydrogen are consumed in a molar ratio of 1:1. It has been found, however, that without any substantial disadvantage wider molar ratios, e.g. ratios of from 1:10 to 10:1 can be applied. Preference is given to ratios carbon monoxide:hydrogen in the range of from 1:0.5 to 1:3.

The process according to the present invention may be carried out in the presence of a solvent. Suitable solvents include carboxylic acids such as acetic acid or propanoic acid; carboxylic acid esters, such as methyl acetate, ethyl acetate, methylpropionate or ethyl propionate (being used as solvent as well as starting material), and cyclic ethers such as tetrahydrofuran, 1,4-dioxane, 1,3-dioxane and the dioxolanes. Also dialkyl ethers used in excess as starting material may be regarded as solvent for the process according to the present invention. Suitable dialkylethers include dimethyl ether, diethyl ether and methyl t-butyl ether.

Other compounds which can be used as solvent in the process according to the present invention include sulphones and sulphoxides. Examples of such compounds are dimethylsulphone, sulfolane, 2-methyl sulfolane, 3-methyl sulfolane, dimethylsulphoxide and diethyl sulphoxide.

Especially good results are obtained when alkanoic acids such as acetic acid are used as solvent. If however a solvent other than an alkanoic acid is used, it may be desirable to carry out the reaction in the presence of small amounts of a strong acid. For example amounts of strong acid of up to 100 equivalents of acid per gram atom of total Group VIII metal, may be added. Suitable strong acids include those which in aqueous solution at 20°C have a $pK_a$ of less than 3.5, for example organic acids such as *p*-toluene sulphonic acid or trifluoromethane sulphonic acid, or mineral acids such as hydrochloric, sulphuric or perchloric acid.

The mild conditions according to the present invention even tolerate the presence of some water in the

reaction medium. Although the presence of water is not preferred, amounts of up to 15%w, based on total solvent, may be present.

The process according to the present invention can be carried out in the liquid phase or in the gaseous phase. Preference is given to a liquid phase which enables a convenient introduction of carbon monoxide and hydrogen into the reaction vessel. If desired, the carbon monoxide and hydrogen can be introduced together into the reaction vessel. The process according to the present invention can be carried out batchwise, semi-continuously or continuously.

The process according to the present invention is also of interest in that it can be integrated with known processes, either for the production of the starting materials (i.e. carboxylic acid esters or the corresponding ethers) or for the conversion of the carboxylic acid esters produced into other products, e.g. by transesterification processes. For instance, when the present process produces ethyl acetate, it can be integrated with a process for the preparation of methyl acetate from acetic acid and methanol using an acidic catalyst. Since the present process produces acetic acid, that compound may be recycled to serve as feedstock for the preparation of methyl acetate. If desired, the present process can also be integrated with a transesterification process, wherein ethylacetate is transesterified with methanol to give methyl acetate (which can be recycled to serve as feedstock for the present process) and ethanol which can either be sold as such or converted into other products such as ethylene. In such a case acetic acid and/or methyl acetate can be removed from the system in an amount equimolar with ethanol produced.

The following examples illustrate the invention.

Example I

The experiment was carried out in a 300 ml magnet-drive autoclave of Hastelloy C (Trade Mark) which contained 25 ml methyl acetate, 25 ml acetic acid, 60 mmol methyl iodide, 0.12 mmol rhodium (III) chloride trihydrate, 1 mmol ruthenium (III) chloride trihydrate and 4 mmol triphenylphosphineoxide. The vessel was pressurized with carbon monoxide (20 bar partial pressure) and hydrogen (40 bar partial pressure). The autoclave was then heated at 160°C and kept at this temperature for 5 hours. After this time the reaction mixture was analysed by gas-liquid chromatography and shown to contain 22.1%w ethyl acetate. On a molar basis the conversion of the starting material was about 90% with an almost 100% selectivity towards the two products ethyl acetate and acetic acid. Only traces (less than 0.5%) of by-products were detected: in particular, no alcohols were detected.

Example II

The method of Example I was repeated except that 0.5 mmol rhodium III chloride trihydrate and only 15 mmol methyliodide were used. After the reaction time was over, the reaction mixture contained 14.6 %w ethyl acetate, and 55% of the methyl acetate had been converted to the desired products. Only traces of by-products were observed.

Example III

The experiment of Example I was repeated except that 0.5 mmol triphenylphosphine sulphide instead of 4 mmol triphenylphosphine oxide was used. After five hours reaction time the conversion of the methyl acetate was 58%. The reaction mixture contained 14.3 %w ethyl acetate.

**Claims**

1. A process for the co-production of carboxylic acids of the general formula $R^1$—COOH and $R^2$—COOH and carboxylic acid esters of the general formula $R^1$—$COOCH_2R^2$ and $R^2$—$COOCH_2R^1$, wherein each of the groups $R^1$ and $R^2$, which may be the same or different, represents an alkyl group having from 1 to 20 carbon atoms which may be substituted by fluorine or chlorine or an aryl, alkaryl or aralkyl group which may be substituted by fluorine or chlorine whilst $R^1$ may also represent a hydrogen atom, in which process a carboxylic acid ester of the general formula $R^1$—$COOR^2$ and/or an ether of the general formula $R^3OR^4$, wherein $R^1$ and $R^2$ are as defined hereinbefore and each of $R^3$ and $R^4$, which may be the same or different, represent an alkyl group having from 1 to 20 carbon atoms which may be substituted by fluorine or chlorine or an aryl, alkaryl or aralkyl group which may be substituted by fluorine or chlorine is reacted with carbon monoxide and hydrogen at elevated temperature and pressure characterised in that the reaction is carried out in the presence of a catalytic system which comprises a ruthenium compound, a further Group VIII metal compound, a compound of the formula $R^5Hal$ or $R^5COHal$, wherein $R^5$ has one of the meanings given above $R^2$ and Hal represents an iodide or bromide atom, and a compound having the formula:

$$YZ[(O)_aR'][(O)_bR''][(O)_cR'''] \qquad I$$

wherein Y represents oxygen, sulphur or selenium, Z represents phosphorus, arsenic or antimony, each of R', R'' and R''' independently represents a hydrogen atom or an optionally substituted alkyl, cycloalkyl or aryl group or R' has this meaning and R'' and R''' together represent an alkylene group, and each of a, b and c independently represents 0 or 1, the reaction mixture being substantially free from other transition metal or Group II iodides or bromides.

2. A process according to claim 1, characterized in that as ruthenium compound is used ruthenium (III) chloride, ruthenium (III) chloride trihydrate, ruthenium (IV) chloride, ruthenium (III) bromide, ruthenium oxide or an organic ruthenium salt or complex.

3. A process according to claim 1 or 2, characterized in that as further Group VIII metal compound is used a rhodium compound such as rhodium oxide, rhodium (III) hydroxide, rhodium (III) chloride, rhodium (III) chloride trihydrate, rhodium (III) bromide, rhodium (III) iodide or an organic rhodium salt of complex, preferably rhodium (III) chloride trihydrate, or a palladium compound such as palladium chloride, palladium chloride dihydrate, palladium bromide, palladium iodide, palladium oxide, or an organic palladium salt or complex, preferably palladium chloride, palladium chloride dihydrate and palladium acetate.

4. A process according to any one of the preceding claims, characterized in that the ruthenium compound and the further Group VIII metal compound are used in a ratio between 50:1 and 1:20, preferably between 10:1 and 1:5.

5. A process according to any one of the preceding claims, characterized in that any optionally substituted alkyl, cycloalkyl or aryl group represented by R', R'' and/or R''' has up to 20 carbon atoms, preferably up to 7 carbon atoms.

6. A process according to 5, characterized in that the compound having the formula

$$YZ[(O)_aR'][(O)_bR''][(O)_cR''']$$

is triphenyl phosphine oxide or triphenyl phosphine sulphide.

7. A process according to any one of the preceding claims, characterized in that the molar ratio of the compounds having formula I, in which Y represents oxygen, to ruthenium is preferably in the range of from 0.01:1 to 20:1, more preferably 0.05:1 to 10:1.

8. A process according to any one of claims 1—6, characterized in that when in the compound having formula I the group Y represents sulphur or selenium and the groups R', R'' and R''' are alkyl, cycloalkyl or aryl groups which have not been substituted with electron attracting groups, the reaction mixture contains 0.01—1 mol of the said compound per gram atom of ruthenium.

9. A process according to any one of the preceding claims, characterized in that the reaction is carried out at a temperature in the range of from 50°C to 200°C, and especially between 125°C and 175°C.

10. A process according to any one of the preceding claims, characterized in that the process is carried out using a pressure between 20 and 100 bar.

11. A process according to any one of the preceding claims, characterized in that an alkanoic acid is used as solvent.

**Patentansprüche**

1. Verfahren zur gleichzeitigen Herstellung von Carbonsäuren der allgemeinen Formel $R^1$—COOH und $R^2$—COOH und Carbonsäureestern der allgemeinen Formel $R^1$—$COOCH_2R^2$ und $R^2$—$COOCH_2R^1$, wobei jede der Gruppen $R^1$ und $R^2$, die gleich oder verschieden sein können, eine Alkylgruppe mit 1 bis 20 Kohlenstoffatomen, die durch Fluor oder Chlor substituiert sein kann, oder eine Aryl-, Alkaryl- oder Aralkylgruppe, die durch Fluor oder Chlor Substituiert sein kann, bedeutet, während $R^1$ auch ein Wasserstoffatom bedeuten kann, wobei ein Carbonsäureester der allgemeinen Formel $R^1$—$COOR^2$ und/oder ein Ether der allgemeinen Formel $R^3OR^4$, wobei $R^1$ und $R^2$ wie oben definiert sind und jedes $R^3$ und $R^4$, die gleich oder verschieden sein können, eine Alkylgruppe mit 1 bis 20 Kohlenstoffatomen, die durch Fluor oder Chlor substituiert sein kann oder eine Aryl-, Alkaryl- oder Aralkylgruppe, die durch Fluor oder Chlor substituiert sein kann, bedeutet, umgesetzt wird mit Kohlenmonoxid und Wasserstoff bei erhöhter Temperatur und erhöhtem Druck, dadurch gekennzeichnet, daß die Reaktion in Gegenwart eines Katalysatorsystems durchgeführt wird, umfassend eine Rutheniumverbindung, eine weitere Verbindung eines Metalls der Gruppe VIII, eine Verbindung der Formel $R^5$Hal oder $R^5$COHal, wobei $R^5$ eine der oben für $R^2$ angegebenen Bedeutungen hat und Hal ein Iod- oder Bromatom bedeutet, und eine Verbindung der Formel

$$YZ[(O)_aR'][(O)_bR''][(O)_cR'''] \qquad I$$

in der Y Sauerstoff, Schwefel oder Selen bedeutet, Z Phosphor, Arsen oder Antimon bedeutet und R', R'' und R''' unabhängig voneinander jeweils ein Wasserstoffatom oder eine gegebenen falls substituierte Alkyl-, Cycloalkyl- oder Arylgruppe bedeuten oder R' diese Bedeutung hat und R'' und R''' zusammen eine Alkylengruppe bedeuten und a, b und c jeweils unabhängig voneinander 0 oder 1 bedeuten und das Reaktionsgemisch im wesentlichen frei ist von anderen Iodiden oder Bromiden von Übergangsmetallen oder von Elementen der Gruppe II.

2. Verfahren nach Anspruch 1, dadurch gekennzeichnet, daß als Rutheniumverbindung Ruthenium-(III)-chlorid, Ruthenium-(III)-chlorid-trihydrat, Ruthenium-(IV)-chloride, Ruthenium-(III)-bromid, Rutheniumoxid oder ein organisches Rutheniumsalz oder ein Komplex verwendet wird.

3. Verfahren nach Anspruch 1 oder 2, dadurch gekennzeichnet, daß als weitere Verbindung eines

Metalls der Gruppe VIII eine Rhodiumverbindung wie Rhodiumoxid, Rhodium-(III)-hydroxid, Rhodium-(III)-chlorid, Rhodium-(III)-chloridtrihydrat, Rhodium-(III)-bromid, Rhodium-(III)-iodid oder ein organisches Rhodiumsalz oder ein Komplex, vorzugsweise Rhodium-(III)-chlorid-trihydrat oder eine Palladiumverbindung wie Palladiumchlorid, Palladiumchlorid-dihydrat, Palladiumbromid, Palladiumiodid, Palladiumoxid oder ein organisches Palladiumsalz oder ein Komplex, vorzugsweise Palladiumchlorid, Palladiumchlorid-dihydrat oder Palladiumacetat verwendet wird.

4. Verfahren nach einem der vorangehenden Ansprüche, dadurch gekennzeichnet, daß die Rutheniumverbindung und die weitere Verbindung eines Metalls der Gruppe VIII in einem Verhältnis zwischen 50:1 und 1:20, vorzugsweise zwischen 10:1 und 1:5 verwendet werden.

5. Verfahren nach einem der vorangehenden Ansprüche, dadurch gekennzeichnet, daß jede gegebenenfalls substituierte Alkyl-, Cycloalkyl- oder Arylgruppe, die durch R', R'' und/oder R''' angegeben ist, bis zu 20 Kohlenstoffatome, vorzugsweise bis zu 7 Kohlenstoffatome, aufweist.

6. Verfahren nach Anspruch 5, dadurch gekennzeichnet, daß die Verbindung der Formel

$$YZ[(O)_aR'][(O)_bR''][(O)_cR''']$$

Triphenylphosphinoxid oder Triphenylphosphinsulfid ist.

7. Verfahren nach einem der vorangehenden Ansprüche, dadurch gekennzeichnet, daß das Molverhältnis der Verbindungen der Formel I, bei denen Y Sauerstoff ist, zu Ruthenium vorzugsweise im Bereich von 0,01:1 bis 20:1, insbesondere 0,05:1 bis 10:1 liegt.

8. Verfahren nach einem der Ansprüche 1 bis 6, dadurch gekennzeichnet, daß, wenn in der Verbindung der Formel I Y Schwefel oder Selen bedeutet und die Gruppen R', R'' und R''' Alkyl-, Cycloalkyl- oder Arylgruppen sind, die nicht mit Elektronen anziehenden Gruppen substituiert sind, das Reaktionsgemisch 0,01 bis 1 Mol dieser Verbindung/g-Atom Ruthenium enthält.

9. Verfahren nach einem der vorangehenden Ansprüche, dadurch gekennzeichnet, daß die Reaktion bei einer Temperatur im Bereich von 50 bis 200°C, insbesondere zwischen 125 und 175°C durchgeführt wird.

10. Verfahren nach einem der vorangehenden Ansprüche, dadurch gekennzeichnet, daß das Verfahren unter Anwendung eines Drucks zwischen 20 und 100 bar durchgeführt wird.

11. Verfahren nach einem der vorangehenden Ansprüche, dadurch gekennzeichnet, daß eine Alkansäure als Lösungsmittel verwendet wird.

**Revendications**

1. Un procédé pour la coproduction d'acides carboxyliques des formules générales $R^1$—COOH et $R^2$—COOH et d'esters d'acides carboxyliques des formules générales $R^1$—$COOCH_2R^2$ et $R_2$—$COOCH_2R^1$, où les groupes $R^1$ et $R^2$, qui peuvent être identiques ou différents, représentent chacun un groupe alcoyle ayant de 1 à 20 atomes de carbone qui peut être substitué par du fluor ou du chlore ou un groupe aryle, alcaryle ou aralcoyle qui peut être substitué par du fluor ou du chlore tandis que $R^1$ peut aussi représenter un atome d'hydrogène, selon lequel un ester d'acide carboxylique de la formule générale $R^1$—$COOR^2$ et/ou un éther de la formule générale $R^3OR^4$, où $R^1$ et $R^2$ sont tels que définis ci-dessus et $R^3$ et $R^4$, qui peuvent être identiques ou différents, représentent chacun un groupe alcoyle ayant de 1 à 20 atomes de carbone qui peut être substitué par du fluor ou du chlore ou un groupe aryle, alcaryle ou aralcoyle qui peut être substitué par du fluor ou du chlore, sont mis à réagir avec de l'oxyde de carbone et de l'hydrogène à température et pression élevées, caractérisé en ce que la réaction est conduite en présence d'un système catalytique qui comprend un composé du ruthénium, un composé d'un autre métal du groupe VIII, un composé de la formule $R^5Hal$ ou $R^5COHal$, où $R^5$ a une des significations indiquées ci-dessus pour $R^2$ et Hal représente un atome d'iode ou de brome, et un composé ayant la formule:

$$YZ[(O)_aR'][(O)_bR''][(O)_cR'''] \qquad I$$

où Y représente de l'oxygène, du soufre ou du sélénium, Z représente du phosphore, de l'arsenic ou de l'antimoine, R', R'', R''' représentent chacun indépendamment un atome d'hydrogène ou un groupe alcoyle, cycloalcoyle ou aryle éventuellement substitué ou R' a cette signification et R'' et R''' représentent ensemble un groupe alcoylène, et a, b et c représentent chacun indépendamment 0 ou 1, le mélange réactionnel étant substantiellement exempt d'iodures ou bromures d'autres métaux de transition ou du groupe II.

2. Un procédé selon la revendication 1, caractérisé en ce que comme composé du ruthénium on utilise du chlorure de ruthénium (III), du trihydrate de chlorure de ruthénium (III), du chlorure de ruthénium (IV), du bromure de ruthénium (III), de l'oxyde de ruthénium ou un sel organique ou complexe de ruthénium.

3. Un procédé selon la revendication 1 ou 2, caractérisé en ce que comme composé d'un autre métal du groupe VIII on utilise un composé du rhodium comme de l'oxyde de rhodium, de l'hydroxyde de rhodium (III), du chlorure de rhodium (III), du trihydrate de chlorure de rhodium (III), du bromure de rhodium (III), de l'iodure de rhodium (III) ou un sel organique ou complexe de rhodium, de préférence du trihydrate de chlorure de rhodium (III), ou un composé du palladium comme du chlorure de palladium, du dihydrate de

chlorure de palladium, du bromure de palladium, de l'iodure de palladium, de l'oxyde de palladium ou un sel organique ou complexe de palladium, de préférence du chlorure de palladium, du dihydrate de chlorure de palladium ou de l'acétate de palladium.

4. Un procédé selon l'une quelconque des revendications précédentes, caractérisé en ce que le composé du ruthénium et le composé d'un autre métal du groupe VIII sont utilisés dans un rapport compris entre 50:1 et 1:20, de préférence entre 10:1 et 1:5.

5. Un procédé selon l'une quelconque des revendications précédentes, caractérisé en ce que tout groupe alcoyle, cycloalcoyle ou aryle éventuellement substitué représenté par R', R'' et/ou R''' a jusqu'à 20 atomes de carbone, de préférence jusqu'à 7 atomes de carbone.

6. Un procédé selon la revendication 5, caractérisé en ce que le composé ayant la formule

$$YZ[(O)_aR'][(O)_bR''][(O)_cR''']$$

est de l'oxyde de triphénylphosphine ou du sulfure de triphénylphosphine.

7. Un procédé selon l'une quelconque des revendications précédentes, caractérisé en ce que le rapport molaire des composés ayant la formule I, dans lesquels Y représente de l'oxygène, au ruthénium est compris de préférence entre 0,01:1 et 20:1, en particulier entre 0,05:1 et 10:1.

8. Un procédé selon l'une quelconque des revendications 1—6, caractérisé en ce que quand dans le composé ayant la formule I, le groupe Y représente du soufre ou du sélénium et les groupes R', R'' et R''' sont des groupes alcoyle, cycloalcoyle ou aryle qui n'ont pas été substitués par des groupes attirant les électrons, le mélange réactionnel contient 0,01—1 mole de ce composé par atome-gramme de ruthénium.

9. Un procédé selon l'une quelconque des revendications précédentes, caractérisé en ce que la réaction est conduite à une température comprise entre 50°C et 200°C et spécialement entre 125°C et 175°C.

10. Un procédé selon l'une quelconque des revendications précédentes, caractérisé en ce qu'on met en oeuvre le procédé en utilisant une pression comprise entre 20 et 100 bars.

11. Un procédé selon l'une quelconque des revendications précédentes, caractérisé en ce qu'on utilise un acide alcanoïque comme solvant.